(19) Europäisches Patentamt
European Patent Office
Office européen des brevets

(11) **EP 1 512 373 B1**

(12) **EUROPEAN PATENT SPECIFICATION**

(45) Date of publication and mention
of the grant of the patent:
**17.02.2010 Bulletin 2010/07**

(51) Int Cl.:
***A61B 5/103*** (2006.01)

(21) Application number: **04255139.0**

(22) Date of filing: **26.08.2004**

(54) **Method for assessing pigmented skin**

Methode zur Beurteilung pigmentierter Haut

Procédé pour l'évaluation de la pigmentation cutanée

(84) Designated Contracting States:
**AT BE BG CH CY CZ DE DK EE ES FI FR GB GR HU IE IT LI LU MC NL PL PT RO SE SI SK TR**

(30) Priority: **28.08.2003 US 650581**

(43) Date of publication of application:
**09.03.2005 Bulletin 2005/10**

(73) Proprietor: **JOHNSON & JOHNSON CONSUMER COMPANIES, INC.**
**Skillman,**
**NJ 08558 (US)**

(72) Inventors:
• **Stamatas, Georgias**
**Somerset NJ 08873 (US)**
• **Kollias, Nikiforos**
**Skillman NJ 08558 (US)**

(74) Representative: **Mercer, Christopher Paul et al**
**Carpmaels & Ransford**
**43-45 Bloomsbury Square**
**London WC1A 2RA (GB)**

(56) References cited:
**US-A- 6 070 092**

• **WAGNER JENNIFER K ET AL: "Comparing quantitative measures of erythema, pigmentation and skin response using reflectometry." PIGMENT CELL RESEARCH / SPONSORED BY THE EUROPEAN SOCIETY FOR PIGMENT CELL RESEARCH AND THE INTERNATIONAL PIGMENT CELL SOCIETY. OCT 2002, vol. 15, no. 5, October 2002 (2002-10), pages 379-384, XP002303193 ISSN: 0893-5785**
• **STAMATAS G N ET AL: "Hyperspectral image acquisition and analysis of skin" PROCEEDINGS OF THE SPIE - THE INTERNATIONAL SOCIETY FOR OPTICAL ENGINEERING SPIE-INT. SOC. OPT. ENG USA, vol. 4959, July 2003 (2003-07), pages 77-82, XP002303194 ISSN: 0277-786X**
• **KOLLIAS N ET AL: "Spectroscopic characteristics of human melanin in vivo." THE JOURNAL OF INVESTIGATIVE DERMATOLOGY. JUL 1985, vol. 85, no. 1, July 1985 (1985-07), pages 38-42, XP002303195 ISSN: 0022-202X**
• **STAMATAS G N ET AL: "Blood stasis contributions to the perception of skin pigmentation" JOURNAL OF BIOMEDICAL OPTICS SPIE USA, vol. 9, no. 2, April 2004 (2004-04), pages 315-322, XP002303196 ISSN: 1083-3668**
• **KOLLIAS N ET AL: "Quantitative assessment of UV-induced pigmentation and erythema." PHOTODERMATOLOGY. FEB 1988, vol. 5, no. 1, February 1988 (1988-02), pages 53-60, XP009039063 ISSN: 0108-9684**

**Description**

**FIELD OF INVENTION**

The present invention relates to the assessment of pigmented skin.

**BACKGROUND OF THE INVENTION**

The chromatic characteristics of skin color arise from the interactions of light (primarily absorption and scattering) with the epidermis and the dermis. The primary light absorbers in skin are hemoglobin and melanin. Most of scattering is attributed to collagen fibers and in pigmented skin to melanosomes. Traditionally, skin redness is considered to arise due to locally elevated concentrations of hemoglobin, whereas skin pigmentation is attributed to melanin.

Human skin is structurally and optically heterogeneous. It consists of two discrete layers, the epidermis and the dermis, each with different biological structure and different optical properties arising from their individual absorbing and scattering constituents. The epidermis is a highly cellular tissue consisting of layers of keratinocytes. It is known that the primary absorber in the epidermis is melanin. It has also been shown (Kollias, et al., (1991) J Photochem Photobiol B 9(2): 135-160) that particulate melanin is a major contributor to epidermal scattering. Melanin is synthesized in specialized organelles, the melanosomes, which are manufactured in the melanocytes that are found at the basal layer of the epidermis. The melanosomes are secreted from the dendritic processes of the melanocytes, and they are phagocytosed by the keratinocytes.

The dermis is essentially an acellular tissue sparsely populated by fibroblasts. It consists primarily of extracellular matrix components (collagen and elastin), blood vessels, and lymphatic vessels. Collagen microfibrils are organized into fibers and the fibers into bundles. These structures constitute the major cause of light scattering in the skin. Hemoglobin is found in the dermal blood supply and is responsible for the red appearance of skin, as in the case of erythema. Skin hemoglobin is confined in networks of arterial and venous plexi that run approximately parallel to the skin surface and in small capillaries that run vertical to the skin surface and reach close to the dermal-epidermal junction in the form of capillary loops. Anatomically, one can distinguish a superficial arterial and venous plexus and a deeper plexus. The capillaries stem from the superficial arterial plexus and empty in the superficial venous plexus. The superficial and deeper plexi are interconnected through smaller vessels. At the deeper level, arteriovenous anastomoses (shunts) provide ways for blood flow to bypass superficial skin layers, thus enabling skin thermal regulation. Circulating erythrocytes in the blood vessels contain high concentrations of hemoglobin. The hemoglobin molecule has four heme groups, which can bind to and deliver oxygen molecules to the tissues. When these binding sites are unoccupied, the molecule is called deoxy-hemoglobin (deoxy-Hb) or reduced hemoglobin. When oxygen molecules occupy these binding sites, it is termed oxy-hemoglobin (oxy-Hb) or oxygenated hemoglobin. Each form of hemoglobin has its own characteristic absorption profile (Kollias, (1995) Clin Dermatol 13(4):361-367).

It follows from the above that the visual perception of skin color is the cumulative result of contributions of various optically active molecules that are found in varying concentrations in the skin. The relative contributions of each chromophore can be evaluated quantitatively by analyzing the remittance spectra of skin tissue. Applicants have discovered that deoxy-hemoglobin not only contributes to erythema, but also surprisingly contributes to perceived pigmentation of the skin. Applicants have accordingly modified the analysis of remittance spectra to take into account this discovery.

**SUMMARY OF THE INVENTION**

The present invention features a method of approximating the relative contribution of melanin to a perceived pigmentation of an area of skin including the steps of: (i) examining such skin with a device including a light source and a reflectance detector, wherein the detector measures the reflectance from such area of skin of light generated by the light source of at a first wavelength of from about 555 nm to about 565 nm, at a second light wavelength of from about 570 nm to about 585 nm, at a third wavelength of from about 620 nm to about 650 nm, and at a fourth wavelength of from about 680 nm to about 750 nm at such area of skin; (ii) determining the first approximate relative contribution of melanin, to such perceived pigmentation by using the calculated absorbance of the third wavelength and the fourth wavelength at such area of skin; and (iii) further determining the approximate relative contribution of melanin by subtracting from the first approximate relative contribution of melanin the approximate relative contribution of deoxy-hemoglobin determined by the absorbance of the first wavelength and the second wavelength at such area of skin.

Other features and advantages of the present invention will be apparent from the detailed description of the invention and from the claims.

## BRIEF DESCRIPTION OF FIGURES

Fig 1a is a graph showing the change in concentration of oxy-hemoglobin at various SSR dosages.

Fig 1b is a graph showing the change in concentration of deoxy-hemoglobin at various SSR dosages.

Fig 1c is a graph showing the change in concentration of melanin at various SSR dosages.

Fig 2 is a graph showing the change in concentration of oxy-hemoglobin, deoxy-hemoglobin, melanin, and scattering at various applied pressures.

Fig 3 is a graph showing that perception of "skin pigmentation" depends on deoxy-hemoglobin concentration in a similar fashion to its dependence on melanin.

Fig 4 is graph showing the change in concentration of oxy-hemoglobin, deoxy-hemoglobin, melanin, and scattering following topical application of 3% $H_2O_2$

## DETAILED DESCRIPTION OF THE INVENTION

It is believed that one skilled in the art can, based upon the description herein, utilize the present invention to its fullest extent. The following specific embodiments are to be construed as merely illustrative, and not limitative of the remainder of the disclosure in any way whatsoever.

Unless defined otherwise, all technical and scientific terms used herein have the same meaning as commonly understood by one of ordinary skill in the art to which the invention belongs.

The present invention relates to a method of approximating the relative contribution of melanin responsible for the perceived pigmentation of an area of skin. What is meant by the term "relative contribution" is either the (i) the concentration of the chromophore (e.g., melanin or deoxy-hemoglobin) at such area of skin or (ii) the percentage of the perceived pigmentation that is a result of the presence of such chromophore at the area of skin. Examples of pigmented areas of the skin include, but are not limited to, freckles, age spots, hyperpigmentation, dark circles, or tanned skin). The methods of the present invention utilize the absorbance of light at various wavelengths. What is meant by the phrase "a light at a wavelength" is radiation band centered at the specified wavelength wherein such band has a full width at half-maximum intensity of less than about 10 nm (e.g., for example less than about 5 nm).

The methods of the present invention can be used to determine the source(s) of perceived pigmentation on areas of the skin. For example, if it is determined that the melanin contributes to the perceived pigmentation, skin lightening/depigmenting agents can be used to treat the area. Examples of such agents include, but are not limited to, ascorbic acid, kojic acid, and soy extracts. If it is determined that the deoxy-hemoglobin contributes to the perceived pigmentation, vasoactive agents can be used to treat the area. Examples of such agents include cortisone and hydrogen peroxide.

Objective quantitative evaluation of skin color using non-invasive instrumentation has been used since the early decades of the 20th century (Brunsting, L. A. and C. Sheard, (1929), J Clin Invest 7(4): 575-592). Since then spectrophotometers have become smaller and simpler in use. Many researchers have used methods based on reflectance measurements that either give "erythema" and "pigmentation" indices based on simple calculations (Diffey, et al., (1984) Br J Dermatol 111:663-672) or calculate tri-stimulus values (L*a*b* scale) that have been adopted by the international committee of standards (CIE) as the preferred method for color measurement (Westerhof, et al., (1986) Photodermatol, 3:310-314). In the latter method, L* and b*, as well as combinations of the two, have been used as "pigmentation" parameters, and a* as the "erythema" parameter. Recent studies have showed that in both methods what is clinically perceived erythema and pigmentation does not correlate linearly with the calculated indices (Takiwaki, et al., (2002), Skin Res Techn 8:78-83 and Wagner et al., (2002), Pigment Cell Res, 15:5:379-384). A more accurate method is to analyze the remitted spectrum to its constituents based on Diffuse Reflectance Spectroscopy (DRS) (Kollias, et al., Clin. Dermatol. 13:36 (1995)). Apparent concentrations of melanin, oxy-Hb, and deoxy-Hb can be extracted from absorption spectra obtained by DRS, thus separating the vascular from the melanin reactions that are responsible for erythema and pigment. DRS measurements are rapid, non-invasive, objective, and quantitative. Instruments that perform DRS can be small, portable, and easy to use.

The absorbance curve was calculated as the logarithm of the ratio of the diffuse reflectance from a non-irradiated site to the diffuse reflectance from an irradiated site. In previous algorithms, pigment was evaluated from the absorbance curve as the slope of the fitted straight line over the wavelength range of 620-720 nm. Then, the absorbance curve was corrected for the pigment absorption and, finally, the oxy-Hb and deoxy-Hb absorption curves were fitted in the range of 550-580 nm, where they exhibit maxima. Such algorithms, however, over-estimate the contribution of melanin and under-estimate the contribution of hemoglobin to the perceived pigmentation.

It has been assumed that deoxy-hemoglobin contributes to the "red" color in skin just like oxy-hemoglobin, afterall, both of these chromophores appear red in vitro. Deoxy-hemoglobin in skin is found in the superficial venous plexus and in particular in the venules. These vessels are large enough (20-50 microns in diameter) so that light (in the yellow-green part of the sepctrum) is almost completely attenuated when it tries to traverse through them. This means that they appear dark (almost black) and slightly reddish. Slight occlusion of blood flow or compression of venules causes an increase or a decrease in the amount of deoxy-hemoglobin resident in the venules and therefore a change in the color of the skin. A surprising result was that these changes in concentration of deoxy-hemoglobin in the skin appear like changes in melanin pigment.

The main reason why deoxy-hemoglobin contributes to a pigmented skin appearance is that the absorption spectrum of deoxy-hemoglobin in the 630-700 nm range is very similar to the absorption spectrum of epidermal melanin, so whatever light is transmitted has the color balance of pigment. Furthermore, the size of the vessels in the superficial venous plexus is such that the transmitted radiation through these vessels is approximately 50% lower than the incident intensity, and, therefore, they appear dark. Moreover, deoxy-hemoglobin is a dominant pigment in normal skin, contributing 30-50% to the perceived concentration of blood and equal in its contribution to that of oxy-hemoglobin and melanin to skin color.

**Device for Reflectance Acquisition**

In one embodiment, the reflectance acquisition can be accomplished by either point spectroscopy or multi-spectral imaging. In the case of point spectroscopy, the system can, in one embodiment, include a light source, a probe, and a reflectance detector. In one embodiment, the light source is a broad-band source that includes wavelengths from about 560 to about 780 nm. Examples of such broadband sources include, but are not limited to, incandescent light sources, metal-halide light sources, halogen lamps, and broad-band light emitting diodes (LEDs). In one embodiment, the light source if a monochromatic or narrow band source (e.g., emitting a wavelength or wavelengths of light substantially within the range of from about 560 to about 780 nm). Examples of such light sources include, but are not limited to, tunable lasers, narrow-band LEDs, and filtered broad-band sources. A combination of two or more of the above light sources can also be used. Examples of the probes include, but are not limited to, a fiber optic bundle (e.g., for contact with the skin site of interest) or an integrating sphere (e.g., with an opening that comes in contact with the site of interest). In the case of an integrating sphere, care should be taken to account for the contribution of specular reflection to the total reflected signal. In the case where a broad-band light source is used, a dispersive element (such as a grading or a monochromator), or a filter (such as a narrow band interference filter or a liquid crystal tunable filter) should be used to filter the light prior to entering the detector (e.g., to filter the light prior to contact with the skin or to filter the reflected light off the skin prior to entering the detector). Examples of detectors include, but are not limited to, a single photodiode, a photodiode array, a CCD array, or a photomultiplier.

In the case of multispectral imaging (e.g., a series of images acquired at a selected few wavelengths of interest), the system can include a light source and an imaging detector. Examples of light sources include, but are not limited to, broadband or narrow band sources capable of illuminating the area of interest or a combination of light sources as discussed above. The detector can be a digital camera, such as a CCD or CMOS. In the case of broad-band light source, the image needs to be filtered before reaching the detector to the appropriate wavelengths. In one embodiment, filtering can be accomplished by placing appropriate dispersive element or filter between the light source and the detector to filter the light entering the detector. In one embodiment for multispectral imaging, orthogonal polarization between the illumination and the detector is used in order to eliminate specular reflection (e.g., glare). Orthogonal polarization can be accomplished by placing a linear polarizer in front of the light source and a second linear polarizer in front of the detector (e.g., the camera). The second linear polarizer can be placed in such a way that its polarization plane is orthogonal to the polarization plane of the first linear polarizer.

**Algorithm for Analysis of Absorbance**

Applicants have discovered a new corrected algorithm for the analysis of reflectance spectra from skin that separates the approximate vascular from the approximate melanin contributions to the perceived erythema and/or pigmentation by taking into account the spectral contribution of deoxy-Hb in the red region of the spectrum (620-700 nm). In one embodiment, the procedure is as follows: (a) the diffuse reflectance spectrum from skin is referenced to uninvolved normal skin of the same individual, (b) the spectrum is fitted with a straight line in wavelength (f($\lambda$)) in the spectral range 620-720 nm (the slope of the straight line is believed to be related to the concentration of melanin in the skin); (c) the straight line that best fits the data is then subtracted from the whole spectrum, which results in a spectrum that is identical with the baseline at wavelengths in the range 620-720 nm and deviating from baseline to shorter wavelengths; (d) the difference spectrum is fitted using the absorption parameters of oxy-hemoglobin and deoxy-hemoglobin at the wavelengths of 560 and 578 nm, which is accomplished by solving a system of two equations and two unknowns, the result is an apparent concentration of oxy-hemoglobin and of deoxy-hemoglobin; (e) the concentration of deoxy-hemoglobin is used to calculate the contribution of this chromophore to the measured slope of the fitted line of step (b);

(f) the apparent concentration of melanin is related to the slope of the line of step (b) corrected for the absorption of deoxy-hemoglobin in this wavelength range given by step (e); (g) an apparent scattering parameter can be calculated from the value of the corrected fitted line of step (f) at an arbitrary selected wavelength in the range of 630 - 820 nm; (h) the corrected fitted line of step (f) is subtracted from the whole spectrum (similarly to step (c)), and (i) the new difference spectrum is fitted using the absorption parameters of oxy-hemoglobin and deoxy-hemoglobin at the wavelengths of 560 and 578 nm (similarly to step (d)), which is accomplished by solving a system of two equations and two unknowns, the result is the corrected apparent concentration of oxy- and of deoxy-hemoglobin.

In one embodiment of the algorithm, the above steps can be condensed to the following equations:

The intercept ($\text{int}_o$) and the slope ($m_o$) of the absorbance spectra in the 620 - 720 nm range can be calculated from the values of the spectrum at the wavelengths 620 nm and 720 nm ($S^{620}$ and $S^{720}$ correspondingly):

$$\text{int}_o = \frac{720 \times S^{620} - 620 \times S^{720}}{720 - 620}, \qquad m_o = \frac{S^{720} - S^{620}}{720 - 620} \qquad (1)$$

The absorbance values at 560 and 578 nm corrected for melanin (initial approximation) are given by:

$$S_c^{560} = S^{560} - (m_o \times 560 + \text{int}_o), \qquad S_c^{578} = S^{578} - (m_o \times 578 + \text{int}_o) \qquad (2)$$

The concentrations of oxy-Hb ($[HbO_2]$) and deoxy-Hb ($[Hb]$) can be calculated from these corrected values of the absorption spectrum and the extinction coefficients of oxy-Hb and deoxy-Hb at 560 nm and 578 nm, $\alpha_{HbO_2}^{560}$, $\alpha_{HbO_2}^{578}$, $\alpha_{Hb}^{560}$, and $\alpha_{Hb}^{578}$:

$$[HbO_2] = \frac{\alpha_{Hb}^{560} \times S_c^{578} - \alpha_{Hb}^{578} \times S_c^{560}}{\alpha_{Hb}^{560} \times \alpha_{HbO_2}^{578} - \alpha_{Hb}^{578} \times \alpha_{HbO_2}^{560}}, \qquad [Hb] = \frac{\alpha_{HbO_2}^{578} \times S_c^{560} - \alpha_{HbO_2}^{560} \times S_c^{578}}{\alpha_{Hb}^{560} \times \alpha_{HbO_2}^{578} - \alpha_{Hb}^{578} \times \alpha_{HbO_2}^{560}} \qquad (3)$$

The approximate relative contribution of oxy-hemoglobin and deoxy-hemoglobin can be used to examine vascular components of the skin and its related skin conditions, such a erythema, acne, inflammation, rosacea, and spider veins.

Finally, the corrected melanin concentration can be calculated given the slope of the deoxy-Hb extinction coefficient in the range 620-720 nm, $Sl_{Hb}^{620-720}$:

$$[melanin] = m_o - [Hb] \times Sl_{Hb}^{620-720} \qquad (4)$$

The corrected values for oxy-Hb and deoxy-Hb concentrations can be calculated from equations (2) and (3) after substituting $m_o$ with the corrected melanin concentration given in equation (4).

The approximate relative contribution of the light scattering can be calculated from:

$$sc = m_o \times \lambda_{sc} + \text{int}_o \qquad (5)$$

where $\lambda_{sc}$ is the chosen wavelength in the range 630-820nm. The approximate relative contribution of scattering can be used to examine dermal collagen and its related skin conditions, such a wrinkles and fine lines.

The above algorithm can be applied to point spectroscopy as well as to hyper- or multi- spectral imaging. Hyperspectral imaging refers to a series of images acquired at different wavelengths, in such a way that a spectrum can be reconstructed for each individual pixel of the image based on the intensity values of the particular pixel throughout the wavelength range of acquisition. Multi-spectral imaging refers to a series of images acquired at a selected few wavelengths of interest.

Before any analysis of the hyper- or multi-spectral images takes place, care should be taken that the spectral images are registered to cancel any motion artifacts that may have occurred during image acquisition. In one embodiment, image registration includes image translation and rotation, but not image dilation, as the latter may compromise the quantitative nature of the algorithm.

In the case of hyper-spectral imaging, the plot of the intensity value of a pixel versus the acquisition wavelength is equivalent to a reflectance spectrum (scaled from 0 to 255 for 8 bit images) of the imaged object at the spatial position of the pixel. The following procedure describes, in one embodiment, how to convert the hyper-spectral spectrum of a pixel to an absorbance spectrum: (a) the percent reflectance spectrum is calculated by taking the ratio of the pixel intensity values to either the maximum allowed intensity (255 for 8 bit images), the reflectance from a white standard, or an image from a wavelength from about 800 to about 900 nm; (b) the estimated specular reflectance is subtracted from the ratio of step (a) (specular reflectance is given by the Fresnel law and for human skin this value is approximately 0.04); and (c) the negative logarithm of the corrected ratio of step (b) plotted against the acquisition wavelengths is the resulting absorbance spectrum.

Once the absorbance spectrum has been calculated, the procedure described above can be used to evaluate the chromophore values at each pixel. After normalization (byte scaling), the values of each chromophore for all pixels can be displayed as separate images, also referred to as "chromophore maps." See, e.g., Stamatas et al., (2003) Proc. SPIE 4959:77-82 for chromophore maps calculated by a previous algorithm.

In one embodiment, a similar "curve-fitting" procedure can be used to calculate apparent values of other chromophores native to human skin or externally applied. Some examples of native pigments not mentioned above include, but are not limited to, methemoglobin (absorbance at 404 nm and 635 nm), bilirubin (absorbance at 460 nm), beta-carotene, and water (absorbance bands at 970 nm, 1100 nm, etc.). Externally applied chromophores include, but are not limited to, materials that absorb in UV, visible, or NIR, such as sunscreens, cosmetic formulations, make-up, lipstick, and topical drugs. Thus, a similar approach can be used to quantify deposition of an ingredient of a topical formulation.

Alternatively to hyper-spectral imaging a small number of acquisition wavelengths can be selected that would result in chromophore maps using the above procedure. This approach is termed multi-spectral imaging. The requirements for selecting the right acquisition wavelengths are as follows: (a) One image is required to be acquired in the wavelength region of the deoxy-hemoglobin maximum at 560 nm and has to have a bandwidth of less than 5 nm at each side, in order to avoid the maxima of oxy-hemoglobin at 555 nm and 578 nm; (b) One image is required to be acquired in the wavelength region of the beta band of oxy-hemoglobin (at 578 nm) and has to have a bandwidth of less than 10 nm at each side; and (c) At least two images are required to be acquired in the wavelength region of 620 nm - 720 nm with the wavelengths being chosen as far from each other as possible to cover evenly the region.

Melanin and scattering values can be calculated from the (c) images as described in the procedure above and oxy- and deoxy-hemoglobin values from a 2x2 system using the intensities of images (a) and (b). To calculate values for the four chromophores mentioned here, a minimum of four images at different wavelengths should be used.

**Clinical Studies**

Twelve healthy individuals with skin photo-types III-IV participated in the study. The source of irradiation was a 500W UVC-filtered Xenon arc solar simulator (Solar Light Co., Philadelphia, PA). The instrument was calibrated right before its use, and the total power of the source was recorded every 3 to 4 hours throughout the day to assure its stability and spectral quality following Colipa (The European Cosmetic, Toiletry, and Perfumery Association) guidelines. Initially the minimum solar simulator radiation (SSR) dose to induce perceptible erythema (MED) was determined on the back of each participant. Clinical erythema was evaluated 24 hours after the irradiation. Following MED determination, each individual was irradiated on the back with SSR doses of 0.7, 1.0, 1.5, 2.1, and 3 MED. The skin reactions were evaluated on days 1, 7, 14, and 21 after exposure. Evaluations included cross-polarized photography, DRS measurements, and clinical assessment of erythema and pigmentation by an experienced dermatologist.

In a second experiment, a pressure cuff was applied to the upper arm of ten healthy volunteers. The applied pressure was set at levels of 0, 20, 30, 40, and 60 mmHg. Measurements were taken 5 min after each pressure level was set to allow for the vasculature to equilibrate. Changes in skin color due to vascular reactions were evaluated visually with a chromameter and with a DRS instrument.

In a third experiment, 2 inch diameter cotton pads soaked in 3% $H_2O_2$ (U.S.P. topical anti-infective) in aqueous solution were applied on the volar forearm of ten healthy volunteers for 1 min. The pads were removed and the skin area was dried with fresh cotton pads. DRS measurements and visible evaluation of the treated sites were performed at 0, 5, 10, 15, and 20 min after removal of the pads.

**Diffuse Reflectance Spectroscopy (DRS)**

The DRS instrument contained a quartz halogen light source (Ocean Optics, Boca Raton, FL), a bifurcated fiber bundle (Multimode Fiber Optics, East Hanover, NJ), an S2000 spectrometer (Ocean Optics, Boca Raton, FL), and a laptop computer (Toshiba Tecra, Irvine, CA). One leg of the fiber bundle was connected to the light source and the other to the spectrometer. Measurements were performed by placing the common end of the fiber bundle gently in contact with skin so as not to perturb the blood content. A reflectance spectrum was acquired in the range of 400-820 nm. Apparent concentrations of hemoglobin and melanin were calculated from the diffuse reflectance spectra. See, e.g., Kollias et al., Photodermatol 5:53-60 (1988). Briefly, the absorbance curve was calculated as the logarithm of the ratio of the diffuse reflectance from a non-irradiated site to the diffuse reflectance from an irradiated site. Pigment was evaluated from the absorbance curve as the slope of the fitted straight line over the wavelength range of 620-720 nm. Then, the curve was corrected for the pigment absorption, and finally, the oxy-Hb and deoxy-Hb absorption curves were fitted in the range of 550-580 nm, where they exhibit maxima, as set forth in Table 1.

Table I

| Chromophore | Absorption curve characteristic |
| --- | --- |
| Melanin | Monotonic increase towards short wavelengths; Approximates linear in the region 600 - 750 nm |
| Oxy-Hemoglobin | Maxima at 415, 540, and 577 nm |
| Deoxy-Hemoglobin | Maxima at 430 and 555 nm |

The reproducibility of the method for calculating apparent hemoglobin concentrations was calculated as the error between measurements, and it was found to be better than 10%. It needs to be noted that for the collection geometry used here, an underestimation of the reflectance at the long wavelengths (red/NIR region) compared to the shorter wavelengths (blue/green region) is anticipated. However, the measurements were always preformed relative to baseline, or to neighboring untreated skin, and, therefore, such artifacts have been normalized.

**Data Analysis**

Linear regressions of the data were calculated using the least square errors algorithm. The goodness of fit is given by the correlation coefficient (R-squared). Statistical significance was calculated using the Student's t-test for paired data distributions.

**UV Irradiation Experiment**

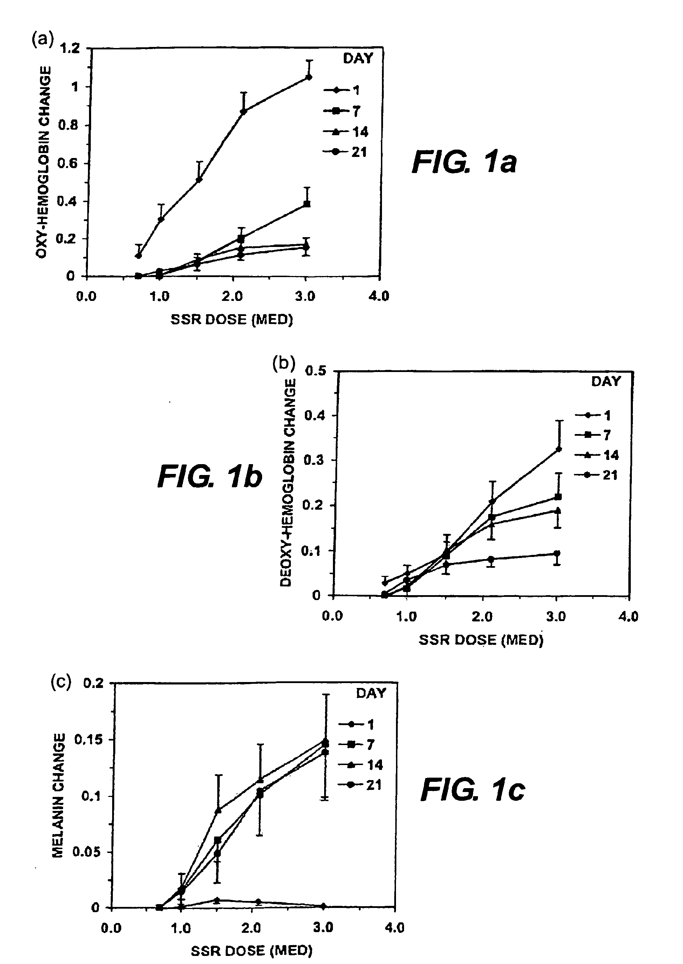

UV-induced erythema was typically evaluated on day 1 after irradiation, while pigmentation was evaluated on day 7. The time course of changes in the concentration of oxy-Hb, deoxy-Hb, and melanin are shown in Figs 1a, 1b, and 1c. On day 1, after irradiation the observed skin reaction was classified as clinical "erythema". The visual observation of erythema correlated well with a dramatic increase in oxy-Hb and deoxy-Hb, as calculated from the spectroscopic data (Figs. 1a and 1b). The observed skin reaction on day 7 was classified clinically as "pigmentation", which correlated with an increase in melanin (Fig. 1c). However, the levels of both hemoglobins were significant on day 7, indicating that there is a strong vascular contribution to the observed reaction (Figs. 1a and 1b). On day 14, the reaction was again classified as "pigmentation", and although the melanin levels remained elevated, the concentration of deoxy-Hb was still above baseline (Fig. 1b). On day 21, the observed pigmentation was almost exclusively due to melanin (Fig. 1c). Although deoxy-Hb is still measurably above baseline, its contribution at 0.1 level was not visibly perceptible.

**Pressure cuff experiment**

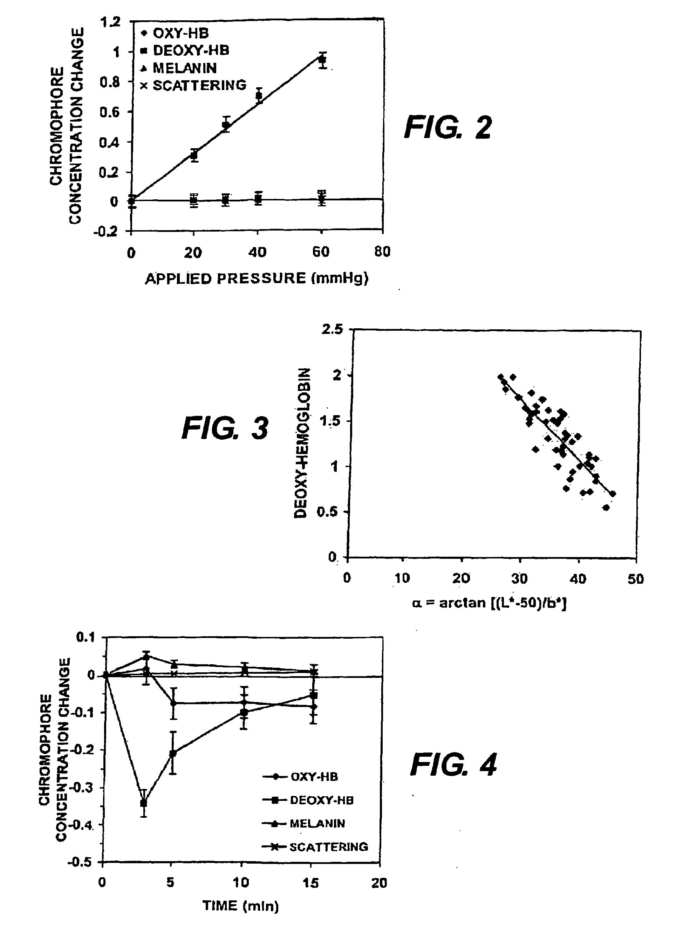

In the pressure cuff experiment, increasing pressure resulted in a reduction of the values of L* and b* as measured by the chromameter corresponding to darker and less yellow color respectively. On the contrary, the value of a* increased corresponding to a more red appearance. Although the color changes were recorded with the chromameter, it is of interest to note that visually the change in skin color was hard to observe. The reason was that the human eye works better in contrast and since the pressure was applied on the whole of the arm, the color change was uniform and, therefore, difficult to detect. DRS analysis showed that the only chromophore that was affected by changing the applied pressure was deoxy-Hb, which increased linearly with pressure (Fig. 2). Oxy-Hb and melanin remained practically unaltered. The characteristic angle, $\alpha = \arctan((L*-50)/b*)$, is a measure of the perceived pigmentation in such a way that when apparent pigmentation increases this parameter decreases. See, e.g., Park, et al., Clinexp Dermatol 24: 315-320 (1999). In the present experiment, the characteristic angle decreased with increasing pressure in all volunteers, indicating that pressure-induced increases in blood stasis can be perceived as increased pigmentation (Fig. 3).

**Hydrogen Peroxide Experiment**

Application of cotton pads soaked in 3% hydrogen peroxide for 1 min induced a decrease in perceptible skin pigmentation that lasted for 10-15 min after removal of the pads. Analysis of DRS spectra showed that deoxy-Hb significantly decreased during the period of blanching (Fig. 4). Oxy-Hb decreased slightly, but within instrument variability (determined to be $\pm$ 0.1 oxy-Hb units). Melanin and dermal scattering remained unchanged at baseline levels.

It is understood that while the invention has been described in conjunction with the detailed description thereof, that the foregoing description is intended to illustrate, and not limit the scope of the invention, which is defined by the scope of the appended claims. Other aspects, advantages, and modifications are within the claims.

**Claims**

1. A method of approximating the relative contribution of melanin to a perceived pigmentation of an area of skin, said method comprising the steps of:

   (i) examining said area of skin with a device comprising a light source and a reflectance detector, wherein said detector measures the reflectance from said area of skin of light generated by said light source of at a first wavelength of from 555 nm to 565 nm, at a second light wavelength of from 570 nm to 585 nm, at a third wavelength of from 620 nm to 650 nm, and at a fourth wavelength of from 680 nm to 750 nm at said area of skin,
   (ii) determining the first approximate relative contribution of melanin to such perceived pigmentation by using the calculated absorbance of said third wavelength and said fourth wavelength at said area of skin; and
   (iii) further determining the final approximate relative contribution of melanin by subtracting from said first approximate relative contribution of melanin the approximate relative contribution of deoxy-hemoglobin determined by the absorbance of said first wavelength and said second wavelength at said area of skin.

2. The method of claim 1, wherein said device comprises a filter device such that the light emitted from said light source is filtered to said first wavelength, said second wavelength, said third wavelength, and said fourth wavelength, optionally prior to measurement by said reflectance detector.

3. The method of claim 1, wherein said reflectance detector is a spectrometer.

4. The method of 1, wherein said reflectance detector is a camera.

5. The method of claim 4, wherein said method is conducted at a plurality of areas of the skin where the absorbances at such areas of skin are determined from the pixels obtained by said camera.

6. The method of claim 5, wherein said relative contribution of melanin or deoxy-hemoglobin at said areas of skin is represented as an image of such areas of skin.

**Patentansprüche**

1. Verfahren zum Annähern des relativen Beitrags von Melanin an einer wahrgenommenen Pigmentierung einer Hautfläche, wobei das Verfahren die Schritte umfasst:

   (i) Untersuchen der Hautfläche mit einer Vorrichtung, die eine Lichtquelle und einen Reflexionsgraddetektor umfasst, wobei der Detektor den Reflexionsgrad von Licht von der Hautfläche misst, welches durch die Lichtquelle bei einer ersten Wellenlänge von 555 nm bis 565 nm, bei einer zweiten Lichtwellenlänge von 570 nm bis 585 nm, bei einer dritten Wellenlänge von 620 nm bis 650 nm und bei einer vierten Wellenlänge von 680 nm bis 750 nm an der Hautfläche erzeugt wird,
   (ii) Bestimmen des ersten ungefähren relativen Beitrags von Melanin an einer solchen wahrgenommenen Pigmentierung durch Verwendung des berechneten Absorptionsgrads der dritten Wellenlänge und der vierten Wellenlänge an der Hautfläche; und

(iii) weiteres Bestimmen des endgültigen ungefähren relativen Beitrags von Melanin durch Abziehen des ungefähren relativen Beitrags von Desoxy-Hämoglobin, bestimmt durch den Absorptionsgrad der ersten Wellenlänge und der zweiten Wellenlänge an der Hautfläche, von dem ersten ungefähren relativen Beitrag von Melanin.

**2.** Verfahren nach Anspruch 1, wobei die Vorrichtung eine Filtervorrichtung umfasst, so dass das aus der Lichtquelle emittierte Licht zur ersten Wellenlänge, zur zweiten Wellenlänge, zur dritten Wellenlänge und zur vierten Wellenlänge gefiltert wird, optional vor der Messung durch den Reflexionsgraddetektor.

**3.** Verfahren nach Anspruch 1, wobei der Reflexionsgraddetektor ein Spektrometer ist.

**4.** Verfahren nach Anspruch 1, wobei der Reflexionsgraddetektor eine Kamera ist.

**5.** Verfahren nach Anspruch 4, wobei das Verfahren durchgeführt wird an einer Vielzahl von Hautflächen, wobei die Absorptionsgrade an solchen Hautflächen aus den durch die Kamera erhaltenen Pixeln bestimmt werden.

**6.** Verfahren nach Anspruch 5, wobei der relative Beitrag von Melanin oder Desoxy-Hämoglobin an den Hautflächen als ein Bild solcher Hautflächen dargestellt wird.

## Revendications

**1.** Procédé pour estimer la contribution relative de la mélanine à une pigmentation perçue d'une zone de peau, ledit procédé comprenant les étapes consistant à :

(i) examiner ladite zone de peau avec un dispositif comprenant une source lumineuse et un détecteur de réflectance, ledit détecteur mesurant la réflectance par ladite zone de peau de la lumière générée par ladite source lumineuse à une première longueur d'onde de 555 nm à 565 nm, à une deuxième longueur d'onde de 570 nm à 585 nm, à une troisième longueur d'onde de 620 nm à 650 nm, et à une quatrième longueur d'onde de 680 nm à 750 nm au niveau de ladite zone de peau,
(ii) déterminer la première contribution relative approximative de la mélanine à cette pigmentation perçue en utilisant l'absorbance calculée de ladite troisième longueur d'onde et de ladite quatrième longueur d'onde au niveau de ladite zone de peau ; et
(iii) déterminer en outre la contribution relative approximative finale de la mélanine en soustrayant de ladite première contribution relative approximative de la mélanine, la contribution relative approximative de la désoxyhémoglobine déterminée par l'absorbance de ladite première longueur d'onde et de ladite deuxième longueur d'onde au niveau de ladite zone de peau.

**2.** Procédé selon la revendication 1, dans lequel ledit dispositif comprend un dispositif de filtre de sorte que la lumière émise par ladite source lumineuse soit filtrée à ladite première longueur d'onde, ladite deuxième longueur d'onde, ladite troisième longueur d'onde et ladite quatrième longueur d'onde, facultativement avant mesure par ledit détecteur de réflectance.

**3.** Procédé selon la revendication 1, dans lequel ledit détecteur de réflectance est un spectromètre.

**4.** Procédé selon la revendication 1, dans lequel ledit détecteur de réflectance est un appareil photo.

**5.** Procédé selon la revendication 4, dans lequel ledit procédé est réalisé au niveau d'une pluralité de zones de peau, les absorbances au niveau de ces zones de peau étant déterminées à partir des pixels obtenus avec ledit appareil photo.

**6.** Procédé selon la revendication 5, dans lequel ladite contribution relative de la mélanine ou de la désoxyhémoglobine au niveau desdites zone de peau est représentée sous forme d'une image de ces zones de peau.

FIG. 1a

FIG. 1b

FIG. 1c

FIG. 2

FIG. 3

FIG. 4

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **Kollias et al.** *J Photochem Photobiol B,* 1991, vol. 9 (2), 135-160
- **Kollias.** *Clin Dermatol,* 1995, vol. 13 (4), 361-367
- **Brunsting, L. A. ; C. Sheard.** *J Clin Invest,* 1929, vol. 7 (4), 575-592
- **Diffey et al.** *Br J Dermatol,* 1984, vol. 111, 663-672
- **Westerhof et al.** *Photodermatol,* 1986, vol. 3, 310-314
- **Takiwaki et al.** *Skin Res Techn,* 2002, vol. 8, 78-83
- **Wagner et al.** *Pigment Cell Res,* 2002, vol. 15 (5), 379-384
- **Stamatas et al.** *Proc. SPIE,* 2003, vol. 4959, 77-82
- **Kollias et al.** *Photodermatol,* 1988, vol. 5, 53-60
- **Park et al.** *Clinexp Dermatol,* 1999, vol. 24, 315-320